# EUROPEAN PATENT SPECIFICATION

(11) **EP 2 939 551 B1**
(45) Date of publication and mention of the grant of the patent: **03.02.2021**
(21) Application number: 13869567.1
(22) Date of filing: 12.12.2013
(51) Int. Cl.: A23L 29/231, A23L 29/238, A23L 29/269, A23L 33/20, A23L 2/40, A23L 2/52, A23L 2/54, A61K 9/00, A61K 47/36, A23L 29/256, A61P 3/04

(54) **WATER-BASED CARBONATED BEVERAGE**
KOHLENSÄUREHALTIGE GETRÄNKE AUF WASSERBASIS
BOISSON GAZEUSE AQUEUSE

(30) Priority: 25.12.2012 JP 2012280430
(43) Date of publication of application: 04.11.2015
(73) Proprietor: Taisho Pharmaceutical Co., Ltd., Tokyo 170-8633 (JP)
(72) Inventor: DOMOTO, Takashi, Tokyo 170-8633 (JP); YAMAJI, Marie, Tokyo 170-8633 (JP); SAKATA, Akane, Tokyo 170-8633 (JP)
(74) Representative: Hoffmann Eitle
(86) International application number: PCT/JP2013/083336
(87) International publication number: WO 2014/103737

(56) References cited:
- EP-A1- 1 488 812
- WO-A1-96/19925
- WO-A1-98/04158
- WO-A1-2012/039598
- JP-A- 2011 000 055
- JP-A- 2011 147 444
- JP-A- 2011 527 189
- KR-A- 20110 060 138
- US-A1- 2007 087 038
- US-A1- 2008 014 315
- US-A1- 2008 286 421
- SHOZO MIYAZAKI: 'Ion Otosei Intelligent Tato no Inai Gel-ka Kino o Fuyo shita Shokuhin Tenkabutsu no Kaihatsu' THE JAPAN FOOD CHEMICAL RESEARCH FOUNDATION KENKYU SEIKA HOKOKUSHO. no. 10, 2004, pages 104 - 108, XP008179138

## Description

### [Technical Field]

The present invention relates to an aqueous carbonated beverage, and is utilizable in the fields of drugs, quasi drugs, foods, and so forth.

### [Background Art]

Leading to metabolic syndrome, obesity is a serious social problem. Effective means for preventing obesity includes dieting: restricting amounts of meals taken. However, dieting cannot be continued for a long term due to hunger caused by the dieting per se in real circumstances. Hence, in order to eliminate hunger, a hunger reducing agent containing a flavor or a flavor compound as the main component (see PTL 1), a cereal food (see PTL 2), an edible phosphoprotein and a metal carbonate (see PTL 3), and the like have been provided.

As one of methods for alleviating such hunger, a method has been reported, which uses agar, for increasing the gel strength of the agar which is immersed in a gastric juice for a certain period (see PTL 4). Nevertheless, this method is hardly said to be highly practical because such hard gel has to be chewed and swallowed. Meanwhile, there have also been reports on a method which uses a gastric raft composition containing a gelling agent (see PTL 5) and a method which uses a gastric raft composition containing a gas producing material capable of producing a nontoxic gas when coming into contact with an aqueous acid (see PTL 6). These have a characteristic of floating in the stomach owing to the influence of the generated gas and are not easily digested by the peristaltic movement of the stomach. However, the compositions have to be taken in a large amount in the form of powder or tablet, and therefore have a problem of administration difficulty. Moreover, when these compositions are dissolved in water, the resulting solutions exhibit an alkalinity due to the influence of the gas generating substance. Hence, there are problems that the storability as a beverage is poor, and a beverage obtained by dissolving such a powder has a taste with a low palatability.

WO 98/04158 A1 describes a carbonated beverage and a process for making it. The beverage is characterized by the addition of a gelling agent such that the final beverage has a light gel texture.

WO 96/19925 A1 describes a gelled, alcohol-free carbonated beverage containing from about 2 . 0 to about 3.0 gas volumes of carbon dioxide, comprising between about 0.0001 % and 0.2 % gellan gum. In one embodiment of the invention, the amount of gellan gum in the beverage is between about 0.0001% and 0.01 %. In another embodiment, the amount of gellan gum in the beverage is between about 0.0101 % and 0.0499 %. In another embodiment, the amount of gellan gum in the beverage is between about 0.05 % and 0.2 %.

US 2008/014315 A1 describes a foam-creating composition containing a dairy composition and a hydrocolloid composition.

US 2007/0087038 A1 relates to a method for the administration of a composition that augments the acidity of gastric fluid such that intra-gastric gelation of the polysaccharide/acid soluble multivalent cation formulation is initiated independently of endogenous acid secretion. The composition includes: (a) at least one of a polysaccharide, modified polysaccharide, or polysaccharide salt, each capable of ionotropic gelation, (b) at least one source of multivalent cations capable of solubilization at an acidic pH, and (c) at least one acid component capable of providing a controlled release of protons sufficient to solubilize the multivalent cations.

KR20110060138 describes a jelly carbonated beverage containing a gelly syrup. The jelly syrup contains 0.155-0.42 of gelling agent formed with gellan gum, guar gum, and LM pectin.

US2008/286421 discloses a foam-creating composition. It identifies pectin as a hydrocolloid composition that may be included in the foam-creating composition.

### [Citation List]

### [Patent Literatures]

[PTL 1] Japanese Unexamined Patent Application Public ation No. 2008-7427
[PTL 2] Japanese Unexamined Patent Application Public ation No. 2007-53929
[PTL 3] Japanese Unexamined Patent Application Public ation No. 2010-94085
[PTL 4] Japanese Unexamined Patent Application Public ation No. 2008-110923
[PTL 5] International Application Japanese-Phase Publ ication No. 2009-530254
[PTL 6] International Application Japanese-Phase Publ ication No. 2005-507409

### [Summary of Invention]

### [Technical Problem]

An object of the present invention is to provide an aqueous carbonated beverage which is a normal aqueous carbonated beverage before drunken, but which turns into a gel by reaction with gastric acid in the stomach after drunken, so that the aqueous carbonated beverage has a characteristic of floating and remaining long in the stomach as a gel.

### [Solution to Problem]

The present inventors have conducted earnest study to achieve the above object. As a result, the inventors have found that an aqueous carbonated beverage comprising any one of 0.01 w/v% or more of a LM pectin, 0.1 w/v% or more of alginic acid or a salt thereof, and 0.001 w/v% or more of gellan gum, and having a pH set to 3.5 to 7.0, is a normal liquid (aqueous carbonated beverage) before drunken, but turns into a gel by reaction with gastric acid in the stomach after drunken, so that the aqueous carbonated beverage has a characteristic of floating and remaining long in the stomach as a gel. Moreover, since the form is a beverage, such an aqueous carbonated beverage is easily taken and can be designed in an acidic to neutral range. Thus, it is possible to design a beverage having a high storability and a taste with a high palatability.

Aspects of the present invention obtained from such findings are as follows.
(1) An aqueous carbonated beverage characterized by comprising any one of:
   0.025 to 5 w/v% of a LM pectin;
   0.3 to 5 w/v% of alginic acid or a salt thereof; and
   0.0025 to 1 w/v% of gellan gum, wherein
   the aqueous carbonated beverage further comprises water and carbon dioxide, and optionally further comprises 0.001 to 5 w/v% of a pH modifier,
   the aqueous carbonated beverage has a pH of 3.5 to 7.0, the aqueous carbonated beverage has a gas volume of 0.5 to 4.0, wherein the gas volume represents a ratio of the volume of carbon dioxide to the volume of liquid solvent in which the carbon dioxide is dissolved under the standard conditions of **101325** Pa (1 atm) and 15.6°C, provided that the volume of the liquid is taken as 1, and the aqueous carbonated beverage is a liquid.
(2) The aqueous carbonated beverage according to (1), wherein the salt of alginic acid is any one of sodium alginate and potassium alginate.
(3) The aqueous carbonated beverage according to (1), wherein the gellan gum is deacylated gellan gum.

### [Advantageous Effect of Invention]

The present invention makes it possible to provide an aqueous carbonated beverage which has a characteristic of turning into a gel by reaction with gastric acid in the stomach after drunken, the gel floating and remaining long in the stomach.

### [Description of Embodiments]

The term "pectin" refers to a water-soluble polysaccharide mainly containing α-1,4-linked polygalacturonic acid, and the compound is extracted from apples and Citrus. The pectin of the present invention may be derived from either apples or Citrus, but has to be a "LM pectin": the ratio of galacturonic acid in the form of methyl ester is less than 50% (galacturonic acid is a constituent sugar of pectins and present in the free acid form or methyl ester form). Incidentally, a pectin with the methyl ester ratio of 50% or more is referred to as a HM pectin, and is not suitable in the present invention because such pectin does not turn into a gel in an acidic range.

An amount of the LM pectin contained (blended) in the beverage is 0.025 to 5 w/v% in order that the gel strength and the administration easiness can be made excellent, and that the gel can be efficiently increased to a sufficient volume in the stomach. Note that, in the present invention, w/v% indicates a ratio of the mass (g) to a total volume of 100 ml.

The term "alginic acid" refers to a polysaccharide contained in seaweeds such as kelps (Laminariaceae) and *wakame* (*Undaria pinnatifida*), and is widely utilized as a thickening stabilizer. Alginic acid has a structure in which mannuronic acid and guluronic acid are linearly polymerized, and is commercially available in the form of a sodium salt, a potassium salt, a calcium salt, an ammonium salt, or the like. In the present invention, either alginic acid or a salt thereof may be used. Any one of sodium alginate and potassium alginate is preferable from the viewpoint of solubility.

An amount of the alginic acid or salt thereof contained (blended) in the beverage is 0.3 to 5 w/v% in order that the gel strength and the administration easiness can be made excellent, and that the gel can be efficiently increased to a sufficient volume in the stomach.

The term "gellan gum" refers to a polysaccharide produced by a microorganism (*Sphingomonas elodea*) extracellularly, and is widely utilized as a thickening stabilizer. Gellan gum is a linear heteropolysaccharide composed of repeating units each consisting of four sugars of glucose, glucuronic acid, glucose, and rhamnose, and has a carboxyl group derived from glucuronic acid. There are two types of gellan gum: deacylated gellan gum and native gellan gum, depending on the presence or absence of an acetyl group and a glyceryl group present in 1-3 linked glucose. In the present invention, any gellan gum may be used. Deacylated gellan gum is preferable from the viewpoint of gel strength.

An amount of the gellan gum contained (blended) in the beverage is 0.0025 to 1 w/v% in order that the gel strength and the administration easiness can be made excellent, and that the gel can be efficiently increased to a sufficient volume in the stomach.

In the present invention, any one of the above-described LM pectin, alginic acid or salt thereof, and gellan gum may be used. From the viewpoint that the gel volume can be further increased in the stomach after drunken as a carbonated beverage, any one of the LM pectin and the alginic acid or salt thereof is preferably used, and the LM pectin is more preferably used.

The term "aqueous carbonated beverage" refers to one obtained by introducing carbon dioxide under pressure into water suitable for drinking, and then adding the resultant to a food, a food additive, or the like. The aqueous carbonated beverage has a gas volume of 0.5 to 4.0. The term gas volume refers to a ratio of the volume of carbon dioxide to the volume of a liquid solvent in which the carbon dioxide is dissolved under the standard conditions (1 atm, 15.6°C), provided that the volume of the liquid is taken as 1.

The aqueous carbonated beverage has a pH of 3.5 to 7.0 from the viewpoint that the aqueous carbonated beverage is a normal aqueous carbonated beverage before drunken but turns into a gel by reaction with gastric acid in the stomach after drunken. In order to keep the pH within the above-described range, a pH modifier may be blended as necessary. The pH modifier is preferably an organic acid. Particularly, citric acid, malic acid, and adipic acid are preferable from the viewpoints that the buffering capacity is high, and that the aqueous carbonated beverage hardly turns into a gel before drunken. An amount thereof in the beverage is 0.001 to 5 w/v%, more preferably 0.01 to 2 w/v% from the viewpoints of buffering capacity and administration easiness. Moreover, for the production within the above-described pH range, a preservative may be blended as necessary. Particularly, the preservative is preferably benzoic acid and benzoic acid salts such as sodium benzoate, and an amount thereof in the beverage is 0.001 to 0.5 w/v%, more preferably 0.005 to 0.1 w/v% from the viewpoints of storability and administration easiness.

The aqueous carbonated beverage can be produced according to a conventionally known method. For example, each component is added to and dissolved in water by mixing. Thereby, a raw solution of the beverage is prepared. Then, the solution is subjected to pH adjustment and sterilization by heating as necessary. After cooling, carbon dioxide gas is incorporated thereinto (carbonation) in such a manner that the gas pressure is within a predetermined range. The resulting solution is filled into a container, followed by a sterilization step. Thus, the aqueous carbonated beverage can be produced.

Note that although the method for producing the carbonated beverage includes a pre-mix method and a post-mix method, any one of the methods may be used in the present invention.

Further, as other ingredients, a vitamin, a mineral, an amino acid and a salt thereof, a crude drug, a crude drug extract, caffeine, royal jelly, a dextrin, or the like may be blended in the aqueous carbonated beverage as appropriate, as long as the effect of the present invention is not impaired. Furthermore, as necessary, an additive such as an antioxidant, a colorant, a flavor, a taste masking agent, a preservative, or a sweetener may be blended as appropriate, as long as the effect of the present invention is not impaired.

### [Examples]

Hereinafter, the present invention will be described in further detail by illustrating Examples, Comparative Examples, and Test Example.

### (Examples and Comparative Examples)

Citric acid monohydrate and sodium benzoate were added to and dissolved in purified water. The pH was adjusted with hydrochloric acid and an aqueous solution of sodium hydroxide. Each of a LM pectin, a HM pectin, sodium alginate, gellan gum, carrageenan, or xanthan gum was dissolved in the solution. Purified water was further added to the solution to make the total amount 25 ml. The pH was finely adjusted with an aqueous solution of sodium hydroxide. Each test solution was filled into a glass bottle and sterilized, and 75 ml of carbonated water or purified water was added thereto immediately before the evaluation. Thus, aqueous beverages of Examples 1 to 8, 10, 11, 13 to 16, 18, and 19 and of Comparative Examples 1 to 6 were obtained.

In Examples 9, 12, and 17, citric acid monohydrate and sodium benzoate were added to purified water, and each of a LM pectin, sodium alginate, or gellan gum was dissolved therein. Purified water was further added to the solution to make the total amount 25 ml. After carbonated water was added thereto, the pH was adjusted with hydrochloric acid and a solution of sodium hydroxide. The total amount was made to be 100 ml.

Each of the aqueous beverages obtained in Examples 1 to 19 was an aqueous carbonated beverage having a gas volume within a range of 0.5 to 4.0 and a total amount of 100 ml. Tables 1 to 5 show the composition and the pH of each aqueous beverage.

**[Table 1]**

| Component name | Unit | Example 1 | Example 2 | Example 3 | Comparative Example 1 | Comparative Example 2 | Comparative Example 3 | Comparative Example 4 |
|---|---|---|---|---|---|---|---|---|
| LM pectin | mg | 500 | - | - | 500 | - | - | - |
| HM pectin | mg | - | - | - | - | 500 | - | - |
| sodium alginate | mg | - | 500 | - | - | - | - | - |
| gellan gum | mg | - | - | 50 | - | - | - | - |
| carrageenan | mg | - | - | - | - | - | 50 | - |
| xanthan gum | mg | - | - | - | - | - | - | 50 |
| citric acid monohydrate | mg | 200 | 200 | 50 | 200 | 200 | 50 | 200 |
| sodium benzoate | mg | 50 | 50 | 50 | 50 | 50 | 50 | 50 |
| hydrochloric acid | mg | adequate amount | adequate amount | adequate amount | adequate amount | adequate amount | adequate amount | adequate amount |
| aqueous solution of sodium hydroxide | mg | adequate amount | adequate amount | adequate amount | adequate amount | adequate amount | adequate amount | adequate amount |
| purified water, total | ml | - | - | - | 100 | - | - | - |
| carbonated water, total | ml | 100 | 100 | 100 | - | 100 | 100 | 100 |
| pH | | 4.1 | 4.3 | 4.4 | 4.1 | 4.1 | 4.1 | 4.1 |

**[Table 2]**

| Component name | Unit | Example 1 | Example 4 | Example 5 | Example 6 | Example 7 | Example 8 | Example 9 |
|---|---|---|---|---|---|---|---|---|
| LM pectin | mg | 500 | 25 | 50 | 100 | 200 | 500 | 500 |
| citric acid monohydrate | mg | 200 | 200 | 200 | 200 | 200 | 200 | 200 |
| sodium benzoate | mg | 50 | 50 | 50 | 50 | 50 | 50 | 50 |
| hydrochloric acid | mg | adequate amount | adequate amount | adequate amount | adequate amount | adequate amount | adequate amount | adequate amount |
| aqueous solution of sodium hydroxide | mg | adequate amount | adequate amount | adequate amount | adequate amount | adequate amount | adequate amount | adequate amount |
| carbonated water, total | ml | 100 | 100 | 100 | 100 | 100 | 100 | 100 |
| pH | | 4.1 | 4.1 | 4.1 | 4.1 | 4.1 | 3.6 | 6.8 |

**[Table 3]**

| Component name | Unit | Comparative Example 5 | Example 2 | Example 10 | Example 11 | Example 12 |
|---|---|---|---|---|---|---|
| sodium alginate | mg | 500 | 500 | 300 | 500 | 1000 |
| citric acid monohydrate | mg | 200 | 200 | 200 | 200 | 200 |
| sodium benzoate | mg | 50 | 50 | 50 | 50 | 50 |
| hydrochloric acid | mg | adequate amount | adequate amount | adequate amount | adequate amount | adequate amount |
| aqueous solution of sodium hydroxide | mg | adequate amount | adequate amount | adequate amount | adequate amount | adequate amount |
| purified water, total | ml | 100 | - | - | - | - |
| carbonated water, total | ml | - | 100 | 100 | 100 | 100 |
| pH | | 4.3 | 4.3 | 4.3 | 3.9 | 6.7 |

**[Table 4]**

| Component name | Unit | Comparative Example 6 | Example 3 | Example 13 | Example 14 | Example 15 | Example 16 | Example 17 |
|---|---|---|---|---|---|---|---|---|
| gellan gum | mg | 50 | 50 | 2.5 | 5 | 10 | 50 | 50 |
| citric acid monohydrate | mg | 50 | 50 | 50 | 50 | 50 | 50 | 50 |
| sodium benzoate | mg | 50 | 50 | 50 | 50 | 50 | 50 | 50 |
| hydrochloric acid | mg | adequate amount | adequate amount | adequate amount | adequate amount | adequate amount | adequate amount | adequate amount |
| aqueous solution of sodium hydroxide | mg | adequate amount | adequate amount | adequate amount | adequate amount | adequate amount | adequate amount | adequate amount |
| purified water, total | ml | 100 | - | - | - | - | - | - |
| carbonated water, total | ml | - | 100 | 100 | 100 | 100 | 100 | 100 |
| pH | | 4.6 | 4.4 | 4.5 | 4.5 | 4.5 | 4.3 | 6.7 |

**[Table 5]**

| Component name | Unit | Example 18 | Example 19 |
|---|---|---|---|
| LM pectin | mg | 2000 | 2000 |
| citric acid monohydrate | mg | 200 | 200 |
| sodium benzoate | mg | 50 | 50 |
| hydrochloric acid | mg | adequate amount | adequate amount |
| aqueous solution of sodium hydroxide | mg | adequate amount | adequate amount |
| carbonated water, total | ml | 100 | 100 |
| pH | | 3.9 | 4.2 |

### (Test Example) State and Volume at the Time of Dripping onto Artificial Gastric Juice

On 100 ml of a Japanese Pharmacopoeia first fluid (first solution for Disintegration Test in Japanese Pharmacopoeia) at 37°C as an artificial gastric juice, 100 ml of each of the aqueous beverages of Examples and Comparative Examples cooled with ice water was dripped. After the incubation at 37°C for 10 minutes, the state was observed, and the total volume was measured. Tables 6 to 10 show the result. Note that, in the tables, "floating" indicates a state where the gelled aqueous beverage was floating on the Japanese Pharmacopoeia first fluid.

**[Table 6]**

| | Unit | Example 1 | Example 2 | Example 3 | Comparative Example 1 | Comparative Example 2 | Comparative Example 3 | Comparative Example 4 |
|---|---|---|---|---|---|---|---|---|
| State before loading onto Japanese Pharmacopoeia first fluid | | liquid | liquid | liquid | liquid | liquid | liquid | liquid |
| State after loading onto Japanese Pharmacopoeia first fluid | | gel | gel | gel | gel | liquid | liquid | liquid |
| State of gel after loading | | floating | floating | floating | no floating | | | |
| Volume after incubation | ml | 272 | 236 | 216 | 203 | 200 | 200 | 199 |

**[Table 7]**

| | Unit | Example 1 | Example 4 | Example 5 | Example 6 | Example 7 | Example 8 | Example 9 |
|---|---|---|---|---|---|---|---|---|
| State before loading onto Japanese Pharmacopoeia first fluid | | liquid | liquid | liquid | liquid | liquid | liquid | liquid |
| State after loading onto Japanese Pharmacopoeia first fluid | | gel | gel | gel | gel | gel | gel | gel |
| State of gel after loading | | floating | floating | floating | floating | floating | floating | floating |
| Volume after incubation | ml | 272 | 206 | 212 | 221 | 236 | 246 | 242 |

**[Table 8]**

| | Unit | Comparative Example 5 | Example 2 | Example 10 | Example 11 | Example 12 |
|---|---|---|---|---|---|---|
| State before loading onto Japanese Pharmacopoeia first fluid | | liquid | liquid | liquid | liquid | liquid |
| State after loading onto Japanese Pharmacopoeia first fluid | | gel | gel | gel | gel | gel |
| State of gel after loading | | no floating | floating | floating | floating | floating |
| Volume after incubation | ml | 200 | 236 | 204 | 228 | 204 |

**[Table 9]**

| | Unit | Comparative Example 6 | Example 3 | Example 13 | Example 14 | Example 15 | Example 16 | Example 17 |
|---|---|---|---|---|---|---|---|---|
| State before loading onto Japanese Pharmacopoeia first fluid | | liquid | liquid | liquid | liquid | liquid | liquid | liquid |
| State after loading onto Japanese Pharmacopoeia first fluid | | gel | gel | gel | gel | gel | gel | gel |
| State of gel after loading | | no floating | floating | floating | floating | floating | floating | floating |
| Volume after incubation | ml | 200 | 216 | 204 | 208 | 206 | 216 | 228 |

**[Table 10]**

| | Unit | Example 18 | Example 19 |
|---|---|---|---|
| State before loading onto Japanese Pharmacopoeia first fluid | | liquid | liquid |
| State after loading onto Japanese Pharmacopoeia first fluid | | gel | gel |
| State of gel after loading | | floating | floating |
| volume after incubation | ml | 212 | 236 |

Unlike the simple aqueous beverage comprising the LM pectin as in Comparative Example 1, the aqueous carbonated beverage comprising the LM pectin as in Example 1 expanded as bubbles of carbon dioxide gas were formed inside the gel. As a result, the total volume was increased, the relative density was decreased, and the aqueous carbonated beverage floated. This was the same for the aqueous carbonated beverages comprising sodium alginate or gellan gum (Examples 2 and 3, compared with Comparative Examples 5 and 6).

Meanwhile, as for the aqueous beverages of Comparative Examples 2 to 4 respectively comprising the HM pectin, carrageenan, and xanthan gum, no gelation was observed after the dripping onto the Japanese Pharmacopoeia first fluid.

Moreover, the gelation and floating were observed when the amount of the LM pectin blended was 0.025 w/v% or more as illustrated in Examples 4 to 7, and when the pH was within the range of 3.6 to 6.8 as illustrated in Examples 8 and 9. As to sodium alginate, the gelation and floating were observed when the blended amount was 0.3 w/v% or more as illustrated in Example 10, and when the pH was within the range of 3.9 to 6.7 as illustrated in Examples 11 and 12. As to the gellan gum, the gelation and floating were observed when the blended amount was 0.0025 w/v% or more as illustrated in Examples 13 to 15, and when the pH was within the range of 4.3 to 6.7 as illustrated in Examples 16 and 17.

As illustrated in Examples 18 and 19, the gelation and floating were observed even in the low-pH solutions comprising a high concentration of the LM pectin.

### [Industrial Applicability]

The present invention makes it possible to provide an aqueous carbonated beverage which is a normal aqueous carbonated beverage before drunken, but which turns into a gel by reaction with gastric acid in the stomach after drunken, so that the aqueous carbonated beverage has a characteristic of floating and remaining long in the stomach as a gel. Therefore, by providing the present invention as drugs, quasi drugs, and foods which are directed to dieting for preventing obesity, these industries are expected to grow.

## Claims

1. An aqueous carbonated beverage comprising any one of:
0.025 to 5 w/v% of a LM pectin;
0.3 to 5 w/v% of alginic acid or a salt thereof; and
0.0025 to 1 w/v% of gellan gum, wherein
the aqueous carbonated beverage further comprises water and carbon dioxide, and optionally further comprises 0.001 to 5 w/v% of a pH modifier,
the aqueous carbonated beverage has a pH of 3.5 to 7.0,
the aqueous carbonated beverage has a gas volume of 0.5 to 4.0, wherein the gas volume represents a ratio of the volume of carbon dioxide to the volume of liquid solvent in which the carbon dioxide is dissolved under the standard conditions of 101325 Pa (1 atm) and 15.6°C, provided that the volume of the liquid is taken as 1, and
the aqueous carbonated beverage is a liquid.

2. The aqueous carbonated beverage according to claim 1, wherein the salt of alginic acid is any one of sodium alginate and potassium alginate.

3. The aqueous carbonated beverage according to claim 1, wherein the gellan gum is deacylated gellan gum.

## Patentansprüche

1. Wässriges kohlensäurehaltiges Getränk, umfassend eines der Folgenden:
0,025 bis 5 Gew./Vol.-% eines LM-Pektins;
0,3 bis 5 Gew./Vol.-% Alginsäure oder ein Salz davon; und
0,0025 bis 1 Gew./Vol.-% Gellan-Gummi, wobei
das wässrige kohlensäurehaltige Getränk ferner Wasser und Kohlendioxid und gegebenenfalls ferner 0,001 bis 5 Gew./Vol.-% eines pH-Modifikators umfasst,
wobei das wässrige kohlensäurehaltige Getränk einen pH-Wert von 3,5 bis 7,0 aufweist,
wobei das wässrige kohlensäurehaltige Getränk ein Gasvolumen von 0,5 bis 4,0 aufweist, wobei das Gasvolumen ein Verhältnis des Volumens von Kohlendioxid zu dem Volumen des flüssigen Lösungsmittels, in dem das Kohlendioxid unter den Standardbedingungen von 101325 Pa (1 atm) und 15,6 °C gelöst ist, darstellt, vorausgesetzt, dass das Volumen der Flüssigkeit als 1 genommen wird, und
wobei das wässrige kohlensäurehaltige Getränk eine Flüssigkeit ist.

2. Wässriges kohlensäurehaltiges Getränk gemäß Anspruch 1, wobei das Salz der Alginsäure eines von Natriumalginat und Kaliumalginat ist.

3. Wässriges kohlensäurehaltiges Getränk gemäß Anspruch 1, wobei der Gellan-Gummi deacylierter Gellan-Gummi ist.

## Revendications

1. Boisson gazeuse aqueuse comprenant l'un quelconque parmi :
0,025 à 5 % en p/v d'une pectine à faible teneur en méthoxy ;
0,3 à 5 % en p/v d'acide alginique ou d'un sel de celui-ci ; et
0,0025 à 1 % en p/v de gomme gellane, dans laquelle
la boisson gazeuse aqueuse comprend en outre de l'eau et du dioxyde de carbone, et comprend facultativement en outre 0,001 à 5 % en p/v d'un agent de modification du pH,
la boisson gazeuse aqueuse présente un pH de 3,5 à 7,0,
la boisson gazeuse aqueuse présente un volume de gaz de 0,5 à 4,0, dans laquelle le volume de gaz représente un rapport du volume de dioxyde de carbone au volume de solvant liquide dans lequel le dioxyde de carbone est dissous dans des conditions standard de 101 325 Pa (1 atm) et 15,6 °C, à condition que le volume du liquide soit considéré comme étant de 1, et
la boisson gazeuse aqueuse est un liquide.

2. Boisson gazeuse aqueuse selon la revendication 1, dans laquelle le sel d'acide alginique est l'un quelconque parmi l'alginate de sodium et l'alginate de potassium.

3. Boisson gazeuse aqueuse selon la revendication 1, dans laquelle la gomme gellane est de la gomme gellane désacylée.
